(19) Europäisches Patentamt
European Patent Office
Office européen des brevets

(11) **EP 4 420 612 B1**

(12) **EUROPEAN PATENT SPECIFICATION**

(45) Date of publication and mention
of the grant of the patent:
**21.01.2026 Bulletin 2026/04**

(51) International Patent Classification (IPC):
***A61B 6/03*** *(2006.01)* ***A61B 6/40*** *(2024.01)*
***A61B 6/42*** *(2024.01)*

(21) Application number: **23187885.1**

(52) Cooperative Patent Classification (CPC):
**A61B 6/032; A61B 6/4007; A61B 6/4014;
A61B 6/4275**

(22) Date of filing: **26.07.2023**

(54) **COMPUTED TOMOGRAPHY SYSTEM AND METHOD FOR PERFORMING A COMPUTED TOMOGRAPHY SCAN**

COMPUTERTOMOGRAFIESYSTEM UND VERFAHREN ZUR DURCHFÜHRUNG EINES COMPUTERTOMOGRAFIESCANS

SYSTÈME DE TOMODENSITOMÉTRIE ET PROCÉDÉ POUR EFFECTUER UN BALAYAGE DE TOMODENSITOMÉTRIE

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**

(43) Date of publication of application:
**28.08.2024 Bulletin 2024/35**

(73) Proprietor: **Siemens Healthineers AG
91301 Forchheim (DE)**

(72) Inventors:
• **Flohr, Thomas
91486 Uehlfeld (DE)**
• **Hofmann, Christian
91058 Erlangen (DE)**
• **Petersilka, Martin
91325 Adelsdorf (DE)**
• **Sunnegaardh, Johan
91325 Adelsdorf (DE)**

(74) Representative: **Siemens Healthineers
Patent Attorneys
Postfach 22 16 34
80506 München (DE)**

(56) References cited:
**WO-A1-2016/081766 DE-B3- 102007 014 829
US-A1- 2021 383 582**

EP 4 420 612 B1

**Description**

The invention relates to a method for performing a computed tomography scan with a static computed tomography system.

Independent of the grammatical term usage, individuals with male, female or other gender identities are included within the term.

In computed tomography (CT) measurements, x-ray attenuation signals along x-rays with different directions are combined to reconstruct images of an examined object of interest. The time it takes to acquire data of one CT measurement from the different directions may be referred to as acquisition time. In many cases, the examined object is moving during acquisition. For example, moving organs, such as the heart, or a patient slightly moving parts of his/her body, e.g., the limbs or the chest due to breathing, may potentially lead to motion artifacts. Therefore, it is desirable to keep the acquisition time as short as possible. On of the main factors that may limit the acquisition speed is an x-ray tube power. In order to achieve a good signal-to-noise ratio, a sufficient amount of signal is required. The accumulated photon energy that reaches the CT detectors from the x-ray tube determines the accumulated signal. Accordingly, for a desired signal-to-noise ratio, the possible acquisition speed is typically proportional to the x-ray tube power. Thus, for a higher acquisition speed, a higher tube power is required. However, increasing the x-ray tube power is typically associated with increased costs and space requirements. In particular for static computed tomography systems with non-rotating x-ray sources and detectors, the power of individual x-ray sources tends to be rather low. For example, the maximum power may be at around 10kW, which is significantly lower than what may be used with rotating systems. Further increasing the power may in many situations be technically difficult or not feasible at all.

US 2021/0383582 A1 discloses a method for a stationary computed tomography system.

It is an object of the invention to provide an alternative to known solutions for performing a computed tomography scan with a static computed tomography system.

This object is met by a method according to claim 1. Further advantages and features result from the dependent claims, the description and the attached figures.

A computed tomography (CT) system is hereby disclosed. The computed tomography system comprises an examination area, a static x-ray source array with x-ray sources that are arranged around the examination area, a detector arrangement with detectors that are arranged around the examination area, and a beam collimator, preferably a circular beam collimator, positioned in a radial direction between the x-ray source array and the examination area. The computed tomography system is configured to acquire computed tomography scan data of an object or subject placed in the examination area by simultaneously activating at least two x-ray sources and measuring the corresponding signals with the detector arrangement and repeating the simultaneous activation multiple times with different x-ray sources of the x-ray source array, respectively, in order to acquire a full scan of the object or subject.

A computed tomography system having a static x-ray source array may be called a static computed tomography system. Hence, herein, the term "static computed tomography system" may refer to a computed tomography system with a static x-ray source array. The static computed tomography system may preferably be a computed tomography system of the fourth generation. A static x-ray source array may be an x-ray source array, wherein the x-ray sources are stationary during operation. In other words, the x-ray sources are not rotated around the examination area. A scan of the examination area may be achieved by selectively activating x-ray sources. For example, the x-ray sources may be selectively activated by electronic switching.

To the knowledge of the inventors of this invention, at least with static x-ray sources, activating two or more sources at once has not been considered as a realistic option in the state of the art. This may be due to activating two or more sources at once can seem disadvantageous and even infeasible, since the signals from two (or more) x-ray sources may interfere with each other, e.g., x-rays from both (or more) x-ray sources may reach the same detector at the same time. In a rotating CT it may be easier to avoid this via collimation with a collimator that moves with the rotating x-ray source. On the other hand, for static CT, a collimator limiting the x-ray beam distribution in a direction perpendicular to an axial direction may be typically thought not to be feasible because the collimator used for one x-ray source might block other x-ray sources of the x-ray source array. However, it has been found that, surprisingly, a beam collimator can still be enough to separate or at least distinguish the signals sufficiently even for a static x-ray source array. In particular, separating the signals may be feasible with the beam collimator even if the collimator comprises an essentially constant opening in a direction perpendicular to the axial direction. This is explained in more detail in later parts of this description.

By activating two or more x-ray sources simultaneously, the total power of the x-ray sources per time unit can be increased. For example, activating two x-ray sources at once can increase the power by the factor 2. This can be particularly advantageous for static CT, since, according to the current state of the art, it is often difficult or even impossible to provide the individual x-ray sources with a power output comparable to the x-ray power output of a rotating CT system. For example, a maximum power of x-ray sources for static x-ray source arrays may be in the range of about 10 kW. With rotating CT systems, typically much higher power values are possible. Furthermore, activating two x-ray sources simultaneously may decrease the overall scan time that is needed. Hence, advantageously, the computed tomography system may enable a better usage of the available power reserve, by providing more x-ray power per time unit, and an improved temporal resolution with the static x-ray source array.

The system is configured to repeat the simultaneous activation multiple times with different x-ray sources of the x-ray source array, respectively. For example, two opposite positioned x-ray sources may be activated simultaneously, and then two other opposite positioned x-ray sources may be activated and so on, until all the x-ray sources are activated and/or until enough data is acquired to reconstruct an image.

In the context of this invention, an axial direction is a direction that is perpendicular to the area that is surrounded by the x-ray sources. The axial direction may also be described herein as z-direction. A radial direction is a direction that is perpendicular to the axial direction. Preferably, the radial direction may be any direction from or to the centre of the examination area that is perpendicular to the axial direction. The radial direction may also be described as x,y-direction or as any direction on the x,y-plane from or to the centre of the examination area.

The examination area is preferably designed such that an object or subject can be placed in the examination area. A subject may, for example, be a human being or animal, such as a patient. An object may, for example, be part of a subject, such as a limb or an organ. The examination area may correspond to a scan field of view. A scan field of view may be an area, every section of which can be reached by at least some of the x-ray beams from all x-ray sources such that the x-ray beams, potentially attenuated by the object or subject, reach at least one of the detectors after going through the examination area. Preferably, the x-ray beams can reach the detectors in a straight line through the examination area.

The x-ray sources of the x-ray source array are arranged around the examination area. Generally, the x-ray sources may be distributed according to any shape around the examination area. Preferably the shape is such that all beam angles required for an CT image reconstruction are covered. Preferably, the x-ray sources may be distributed at least approximately on a circle. A circular distribution may be advantageous for making a reconstruction more efficient and/or easier. For example, the circle may have the form

$$\{(R_f \cos(\alpha), R_f \sin(\alpha), 0) | \alpha \in [0, 2\pi)\},$$

where $R_f$ is the radius of the circle. Preferably, the orientation of $R_f$ is parallel to the orientation or the radial direction(s) and perpendicular to the axial direction. Preferably, the centre of the circle corresponds to the centre of the examination area. For example, the x-ray source array may comprise a number of x-ray sources in the order of magnitude of a few hundred or several hundred. For example, more than 100 x-ray sources may be provided. The x-ray sources may be any kind of x-ray sources suitable for CT. For example, the x-ray sources may be x-ray tubes and/or field emitter-based x-ray sources. X-ray tubes may, for example, be heat-based x-ray tubes or field emitter-based x-ray tubes.

Generally, the detectors may be distributed according to any shape around the examination area. Preferably the shape is such that all beam angles required for an CT image reconstruction are covered. Preferably, the detectors may be distributed at least approximately on a circle and/or on a cylinder around the examination area. A cylinder may mean that the detectors are not only distributed in a circle but are also distributed in an axial direction. This may be advantageous to allow detecting beams that are not only in a plane. Preferably, the cylindrical axis goes through the centre of the examination area. Preferably, the cylindrical axis is parallel to an axial direction. For example, the cylinder may have the form

$$\{(R_d \cos(\delta), R_d \sin(\delta), z) | \delta \in [0, 2\pi), z \in [z_{dl}, z_{dh}]\}.$$

$R_d$ is the radius of the cylinder and $z$ ranging between $z_{dl}$, $z_{dh}$. Determines the height and the position of the cylinder in the axial direction. Preferably, the orientation of $R_d$ is parallel to the orientation or the radial direction(s) and perpendicular to the axial direction. The detector arrangement may preferably be a static detector arrangement. A static detector arrangement may be designed such that it does not rotate around the examination area during a CT scan.

The beam collimator is positioned in the radial direction between the x-ray source array and the examination area. The beam collimator may be adapted to the shape of the distribution of the x-ray sources. Preferably, the beam collimator may be a circular beam collimator. The circle shape of the circular beam collimator may correspond to a circular arrangement of the x-ray sources. The collimator may comprise an opening essentially going around the examination area. For example, the opening may be designed to correspond to the circular shape of the beam collimator and/or the circular distribution of the x-ray sources. The beam collimator may be designed to limit an extent of x-ray beams along the axial direction. The beam collimator may comprise two parts that are offset with respect to each other in the axial direction. The two parts may preferably be two circular parts. The two circular parts may preferably have an essentially identical radius with respect to each other. For example, the two circular parts may be described by the equation

$$\{(R_c \cos(\gamma), R_c \sin(\gamma), z) | \gamma \in [0, 2\pi), z = \{z_{cl}, z_{ch}\}\}.$$

$R_c$ is the radius of the circular parts and $z = \{z_{cl}, z_{ch}\}$ are the two positions of the two circular parts in the axial direction. $\{z_{cl}, z_{ch}\}$ may define an upper and lower end in the axial direction of the opening of the beam collimator. Preferably, the beam collimator is a static beam collimator. A static beam collimator may be designed such that it does not rotate around the examination area during a CT scan. Optionally, the opening of the beam collimator may be adaptable. For example, the system may be configured to adapt the opening based on the size and/or form of the object or subject. The system may be configured to adapt the size of the opening. The system may be configured to adapt the size of the opening at least in the axial direction. For example, a small object or subject may only require a smaller scan field of view corresponding to a smaller examination area. Hence, the system may be configured to reduce the size of the opening in the axial direction for a small object or subject. This may allow to regulate that beams from individual x-ray sources are more restricted, leading to less signal overlap.

According to an embodiment, the detector arrangement and the x-ray source array are offset with respect to each other in an axial direction perpendicular to the radial direction, wherein an opening of the beam collimator is offset with respect to both, the x-ray source array and the detector arrangement, such that it is positioned between the x-ray source array and the detector arrangement in the axial direction. Advantageously, having an offset between the detectors and the x-ray source may be useful to ensure that backside of the detectors is not blocking the way of the x-ray beams that are aimed at the examination area. While it may generally be feasible under some circumstance, to have the x-ray sources and the detectors at the same axial position, e.g. by leaving space between the detectors for the beams, it is far more advantageous to have an offset. For Example, a "closed" arrangement of detectors may advantageously allow for a better image reconstruction. An offset of the beam collimator may be advantageous in allowing to block off at least some of the beams in the x,y-plane due to the resulting geometry between x-ray source examination area and detectors. Hence, the offset may allow for fewer beams of different x-ray sources to reach the same detector. Accordingly, a separation between beams from simultaneously activated x-ray sources may be easier to achieve.

According to an embodiment, the detector arrangement is offset with respect to the x-ray source array in the radial direction such that, in the radial direction, the detector arrangement is positioned closer to the examination area than the x-ray source array. Hence, corresponding to above equations for the distribution of the x-ray sources and the detectors, it may be $R_f > R_d$. In combination with the beam collimator this embodiment may be particularly advantageous to allow for a separation of the beams from the two x-ray sources that are activated simultaneously. Additionally and/or alternatively, the beam collimator is offset with respect to the x-ray source array in the radial direction such that, in the radial direction, the beam collimator is positioned closer to the examination area than the x-ray source array. Preferably, in the radial direction, the beam collimator is closer to the detector arrangement than to the x-ray source array. Particularly preferred, the beam collimator is offset with respect to the detector arrangement in the radial direction such that, in the radial direction, the beam collimator is positioned closer to the examination area than the detector arrangement. This particular variation, in combination with the offset in the axial direction, may be advantageous in enabling a further improved separation of two beams of simultaneously activated x-ray sources. Preferably, in the radial direction, an offset between the detector arrangement and the beam collimator is smaller than an offset between the x-ray source array and the detector arrangement.

According to an embodiment, the x-ray source array, the x-ray sources that are to be activated simultaneously, and the beam collimator are arranged such that, during a scan, none of the detectors simultaneously receives beams from two different x-ray sources that both go through the examination area. Generally, beam collimation in the x,y-plane is only possible to a limited extend, e.g. indirectly via an offset of the beam collimator in the axial direction. This may lead to a large illuminated detector channel range. However, the range of the illuminated detector channel that is illuminated by beams going through the examination area may be smaller. It has been found, that an image reconstruction can still work rather well, as long as a projection of the examination area by beams from one x-ray source does not cover the projection of the examination area by beams from another x-ray source that is activated simultaneously. In other words, the detectors that are used to reconstruct data from beams of one x-ray source, i.e. beams that go through the examination area, do not simultaneously receive beams from another x-ray source that also go through the examination area. Hence, as long as the object or subject is restricted to be inside the examination area, only non-attenuated beams from the other x-ray source are received simultaneously.

According to the invention, a method for performing a computed tomography scan with a static computed tomography system is provided. The computed tomography system comprises an examination area, an x-ray source array with x-ray sources that are arranged around the examination area, a detector arrangement with detectors that are arranged around the examination area, and a beam collimator, in particular a circular beam collimator, positioned in a radial direction between the x-ray source array and the examination area. The computed tomography system may in particular be the computed tomography system as described herein, e.g. according to any embodiment of the computed tomography system as described herein. The method comprises the following steps:

(a) simultaneously activating at least two x-ray sources and measuring the corresponding signals with the detector arrangement in order to acquire computed tomography scan data of an object or subject placed in the examination area;

(b) repeating step (a) multiple times with different x-ray sources of the x-ray source array, respectively, in order to acquire a full scan of the object or subject.

All features and advantages of the system may be adapted to the method and vice versa. In particular, any embodiment of the computed tomography system as described herein may be configured to carry out the method according to any embodiments as described herein.

Preferably, for performing step (b) neither the x-ray source array, nor the detector arrangement nor the beam collimator are rotated around the examination area. For example, step (b) may be repeated such that step (a) is repeated with at least two x-ray sources that are next to the previous at least two x-ray sources, respectively. However, another scheme of repetition is also conceivable.

According to an embodiment, more than two x-ray sources are activated simultaneously, wherein the x-ray sources are activated such that the simultaneously activated x-ray sources are spaced equally on the x-ray source array around the examination area. In other words, spaced equally may mean that the smallest distance from one activated x-ray source to the nearest simultaneously activated x-ray source is the same across all simultaneously activated x-ray sources. For example, assuming $N$ simultaneously activated x-ray sources and a circular arrangement of the x-ray sources, the x-ray sources may be $360°/N$ apart. Advantageously, if the simultaneously activated x-ray sources are spaced equally, a particularly good distinction between the signals can be achieved. Preferably, the x-ray sources may be activated such, that the spacing between the activated x-ray sources is always essentially the same across all repetitions of step (b). Preferably, the number $N$ may be chosen according to the size of the examination area or a corresponding scan field of view. In particular the number $N$ may be greater for a smaller size of the examination area and smaller for a greater size of the examination area. The size of the examination area may be defined by the maximum distance of an outer surface of the object or subject in the examination area to the centre of the x-ray source array and/or of the examination area. Optionally, the size of the examination area may be adapted dynamically based on the size of the object or subject. For example, when examining a small object, the size of the examination area may be set to be smaller and, when examining large object, the size of the examination area may be set to be greater than for the small object.

According to an embodiment, exactly two x-ray sources are activated simultaneously. Two x-ray sources may allow a relatively large spacing, compared to more x-ray source, thus making it easier to distinguish between beams from the simultaneously activated x-ray sources. On the other hand, two x-ray sources may still allow for a significantly increased power output per time unit, e.g. twice the power output compared to only a single x-ray source activated at once. The two x-ray sources may be $180° \pm \mu$, where $\mu$ is an angle defining a deviation from $180°$. The angle $\mu$ activated may be set depending on the system geometry. The system geometry may comprise the examination area and the size of the examination area. The system geometry may comprise size, form and position of the x-ray source array, the detector arrangement and/or the beam collimator.

According to an embodiment, an angle between the two simultaneously activated x-ray sources on the x-ray source array is essentially $180°$. An angle of essentially $180°$ can be advantageous because, typically, the best separation between the two simultaneous beams may be achievable with this angle.

According to an embodiment, a smallest angle between the two simultaneously activated x-ray sources on the x-ray source array is smaller than $180°$, preferably smaller than $176°$, more preferably between $175°$ and $90°$. A smallest angle may be understood such that it describes the smallest distance between the two x-ray sources, following along around the x-ray source array. Deviating from a setting where the x-ray sources are $180°$ apart can be advantageous in allowing to have a better time resolution. Generally, when the x-ray sources are exactly opposite the signal that can be acquired simultaneously by both x-ray sources is very similar. In particular, the corresponding x-ray beams going through the examination area are just taking the essentially opposite path. However, when the x-ray sources are not exactly opposite each other, different directions may be measured simultaneously, thus allowing for a better correction of motion. The smallest angle between the two x-ray sources being $90°$ may be particularly advantageous. With an angle of $90°$ the movement correction may be particularly efficient. On the other hand, depending on the system geometry, a separation of only $90°$ may be too close such that the separation of the signals may be difficult or even impossible. Hence, it may be beneficial to choose a compromise between below $180°$ and $90°$. The compromise may be chosen dependent on the actual system geometry and/or object or subject to be examined.

According to an embodiment, x-ray sources are activated simultaneously such that none of the detectors simultaneously receive beams from two different x-ray sources that both go through the examination area. Hence, advantageously, each detector at most receives signals from one x-ray source that goes through the examination area. On the other hand, signals that do not go through the examination area may still be received. Not going through the examination area may be understood such, that the signals are not attenuated by the object or subject. Assuming that only air or, optionally, a constant material that is independent of the object or subject, is present in the area outside the examination area that is relevant for the path of the beams received by the detectors the beams outside the examination area will be not attenuated or attenuated with a constant number. Accordingly, in this embodiment, the data measured by the respective detector may be a sum of data corresponding to a projection through the examination area from one x-ray source and unattenuated (or constant) data from other simultaneously activated x-ray sources. This superposition of x-rays may be relatively easy to correct when reconstructing the CT image.

According to an embodiment, the method comprises a further step of acquiring data with the object or subject not being in the examination area to acquire unattenuated data, and another further step of using the unattenuated data to adjust the data acquired with the object or subject being in the examination area. Accordingly, the unattenuated data may be used to determine calculate which influence the x-rays not going through the examination area may have on the total received signal. The acquisition of the unattenuated data may be used as a calibration step. Advantageously, the unattenuated data may be acquired once and used for multiple scans of objects or subjects.

According to an embodiment, the unattenuated data is acquired for each x-ray source individually. Acquiring the unattenuated data for each x-ray source individually may in particular mean acquiring the unattenuated data for each x-ray source separately. Hence, the unattenuated data may be acquired for one x-ray source after the other. Therefore, the interference between the x-ray sources may be reduced when acquiring the data. Accordingly, a particularly reliable allocation of received signals to individual x-ray sources may be possible.

According to an embodiment, the adjusting is performed for each detector by subtracting the unattenuated data that does not cross the examination area before being detected by the detector from the data acquired with the object or subject being in the examination area. For image reconstruction, it is advantageous to only have data from one "main" x-ray source for each detector at once. Hence, according to tis embodiment the data from one or more other x-ray sources can be subtracted from the received signal. Hence, for a specific detector, the part of the data that originates from beams from other x-ray sources that are active at the same time as the "main x-ray source" can be subtracted essentially leaving only data from the main x-ray source. This subtraction can be possible due to the signals form the other x-ray sources being essentially unchanged by the object or subject because their respective beams are not going through the examination area before entering said specific detector.

For image reconstruction a filtered backprojection algorithm such as the one explained by Feldkamp, I., Davis, L. & Kress, in J. Practical cone-beam algorithm Journal of the Optical Society of America A: Optics and Image Science, and Vision, 1984, 1, 612-619, may be used. This algorithm can preferably be applied when the object or subject is completely within the examination area.

Optionally, a noise reduction algorithm may be applied. For example, the noise reduction may comprise a low pass filtering. Noise reduction may be advantageous for alleviating noise originating from unwanted signal contributions. Unwanted signal contributions may be the contributions from the other x-ray sources that do not pass the examination area, e.g. as described above. Due to these signals being unattenuated, their noise level may still be relatively high compared to the actual signal, in particular when the examined object or subject is large and/or causes a strong attenuation. Since noise is typically a random effect, the noise cannot always be eliminated by subtracting the acquired unattenuated data. Accordingly, the noise remaining after the subtraction may still be significant. Here the noise reduction may help to further improve the reconstructed image by removing and/or reducing the noise.

In the case where individual detectors receive signals from multiple x-ray sources that all go through the examination area, or where the object or subject extends outside the examination area, an image reconstruction may be possible by an iterative approach. For example, the method explained by Kunze, H., Härer, H., Stierstorfer K., in Iterative extended field of view reconstruction, Proceedings of SPIE - The International Society for Optical Engineering, 2007, may be adapted to be applied.

A computer program is hereby disclosed, comprising instructions which, when the program is executed by a control unit of a computed tomography system, cause the computed tomography system to carry out the method as described herein. The computed tomography system may preferably be a system as described herein. All features and advantages of the system and of the method may be adapted to the computer program and vice versa.

The embodiments described herein may be combined with each other unless indicated otherwise.

The accompanying drawings illustrate various exemplary embodiments and methods of various aspects of the invention.

Fig. 1 shows a computed tomography system;

Fig. 2 shows a flow diagram of a method for performing a computed tomography scan with a static computed tomography system according to an embodiment of the invention;

Fig. 3 a schematic representation of an axial view of a computed tomography system;

Fig. 4 shows a schematic representation of a side view of the computed tomography system according to the same example as shown in figure 3;

Fig. 5 shows a schematic representation of an axial view of a computed tomography system, wherein two x-ray sources are activated simultaneously;

Fig. 6 shows a schematic representation of an axial view of the computed tomography system, according to the example shown in figure 5 while also illustrating some geometrical relations;

Fig. 7 shows a plot of projected z-coordinates derived from figure 6 for two simultaneously activated x-ray sources;

Fig. 8 shows a plot of projected z-coordinates for three simultaneously activated x-ray sources; and

Fig. 9 shows a plot of projected z-coordinates for three simultaneously activated x-ray sources with a smaller scan field of view than the scan field of view of figure 8.

Similar elements are designated with the same reference signs in the drawings.

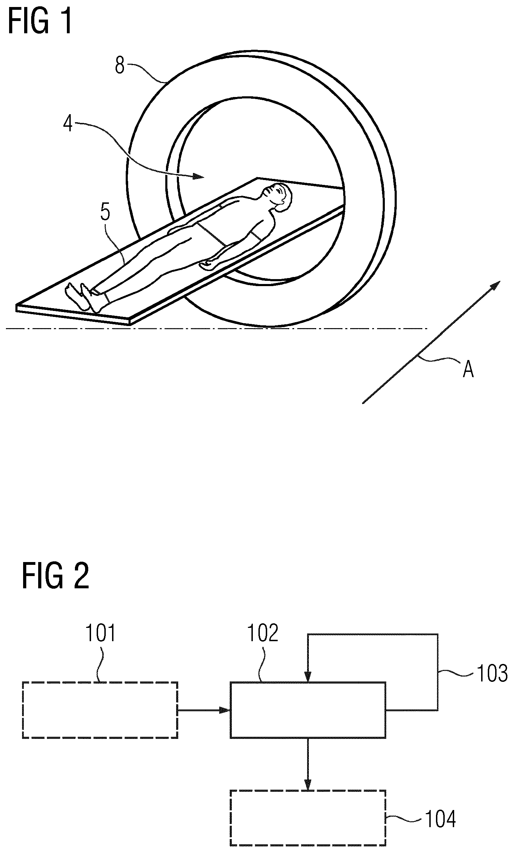

Figure 1 shows a computed tomography system. The computed tomography system comprises an examination area 4. The computed tomography system further comprises a static x-ray source array 1 with x-ray sources 11 that are arranged around the examination area 4, a detector arrangement 2 with detectors 21 that are arranged around the examination area 4, and a circular beam collimator 3 that is positioned in a radial direction between the x-ray source array 1 and the examination area 4. In this embodiment, the array 1, the detector arrangement 2 and the beam collimator 3 are part of a CT ring 8. For example, a subject 5, such as a patient, or part of a subject 5, i.e. an object, may be positioned inside the examination area 4, e.g. by driving the subject 5 into the examination area 4 along the axial direction A via a patient table. The computed tomography system is configured to acquire computed tomography scan data according to an embodiment of a method as described herein. An example of a corresponding method is shown in Figure 2.

Figure 2 shows a flow diagram of a method for performing a computed tomography scan with a static computed tomography system according to an embodiment of the invention. The computed tomography system may, for example, be a system as shown in figure 1. As a first step 102, at least two x-ray sources 11 are activated simultaneously and the corresponding signals are measured with the detector arrangement 2 in order to acquire computed tomography scan data of the subject 5 or part of the subject 5 that is placed in the examination area 4. In a further step 103, the first step 102 is repeated multiple times with different x-ray sources 11 of the x-ray source array 1, respectively, in order to acquire a full scan of the subject 5. Optionally, the method may comprise a further step 101 of acquiring data with the object or subject 5 not being in the examination area 4 to acquire unattenuated data. Preferably, the unattenuated data may be acquired for each x-ray source 11 of the x-ray source array 1 individually, in particular by acquiring the unattenuated data for each x-ray source 11 separately, i.e. one after the other. This further step 101 may preferably be performed before the other steps. However, optionally steps 102 and 103 may also be performed before this step 101. Preferably, this step 101 may be performed once and then be used for multiple performances of the method, i.e. for multiple applications of the computed tomography system. In this optional variant, a further step 104 is provided, wherein the unattenuated data is to adjust the data acquired with the object or subject 5 being in the examination area 4. Preferably, the adjusting may be performed for each detector 21 by subtracting the unattenuated data that does not cross the examination area 4 before being detected by the detector 21 from the data acquired with the object or subject 5 being in the examination area 4.

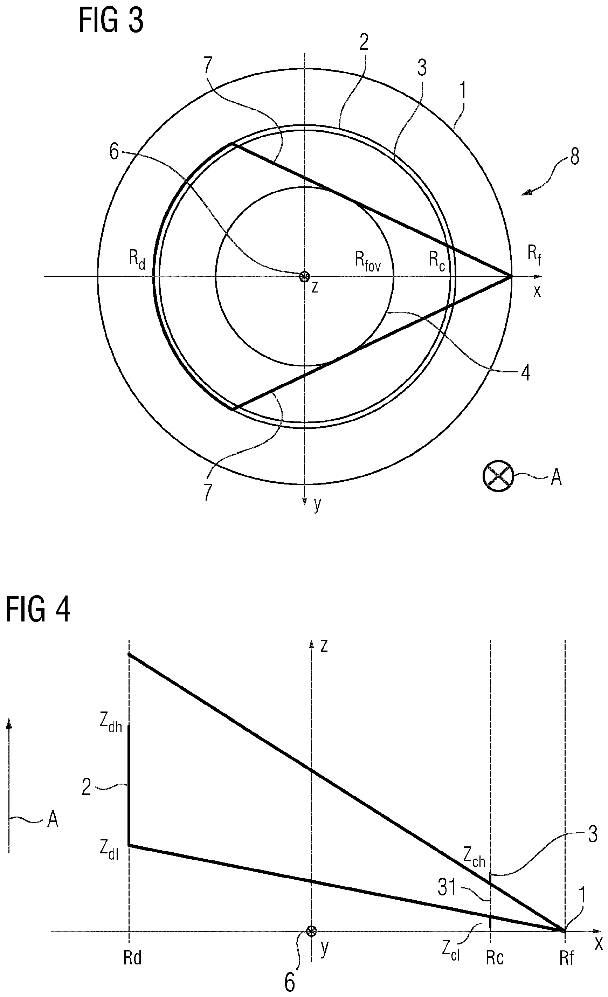

Figure 3 shows a schematic representation of an axial view of the CT ring 8. The plane of view corresponds to the x and y directions. Figure 4 shows a schematic representation of a side view of the CT ring 8 according to the same example. The z direction corresponds to the axial direction A. A static x-ray source array 1 with x-ray sources 11, a detector arrangement 2 with detectors 21, and a beam collimator 3 are arranged around the examination area 4. The x-ray source array 1, the beam detector arrangement 2, the beam collimator 3, and the examination area 4, are all arranged circularly around a common centre 6. The circular beam collimator 3 is positioned in a radial direction between the x-ray source array 1 and the examination area 4. In this embodiment, the circular beam collimator 3 is also positioned in a radial direction between the detector arrangement 2 and the examination area 4. The radial direction is any direction propagating perpendicular to the axial direction A, i.e. in the x,y-plane, from or to the centre 6. The radial positions of the static x-ray source array 1, the detector arrangement 2, the beam collimator 3, and the examination area 4 are designated by $R_f$, $R_d$, $R_c$, $R_{fov}$, respectively. The x-ray sources 11 of the x-ray source array 1 are distributed on a circle defined by

$$\{(R_f \cos(\alpha), R_f \sin(\alpha), 0) | \alpha \in [0, 2\pi)\}.$$

The detectors 21 of the detector arrangement 2 are distributed on a cylinder defined by

$$\{(R_d \cos(\delta), R_d \sin(\delta), z) | \delta \in [0, 2\pi), z \in [z_{dl}, z_{dh}]\}.$$

The beam collimator 3 can be defined by the upper ($z_{ch}$) and lower end of an opening 31 of the beam collimator 3 ($z_{cl}$). The examination area 4 can be defined by a circle

$$\{\left(R_{fov}\cos(\beta), R_{fov}\sin(\beta), 0\right)|\ \beta \in [0,2\pi)\}.$$

In this embodiment, the detector arrangement 2 is offset with respect to the x-ray source array 1 in the radial direction such that, in the radial direction, the detector arrangement 2 is positioned closer to the examination area 4 than the x-ray source array 1. Hence, $R_d$ is smaller than $R_f$, in this embodiment. In this embodiment, the examination area 4 corresponds to a scan field of view. The scan field of view is defined such that the detectors 21 of the detector arrangement 2 are fully illuminated for this scan field of view across the axial direction A. Here, the scan field of view can be defined by outer beams 7 from the corresponding x-ray sources 11 of the x-ray source array 1.

Furthermore, as can be seen in figure 4, the detector arrangement 2 and the x-ray source array 1 are offset with respect to each other in the axial direction A. The opening 31 of the beam collimator 3 is offset with respect to both, the x-ray source array 1 and the detector arrangement 2, such that it is positioned between the x-ray source array 1 and the detector arrangement 2 in the axial direction A.

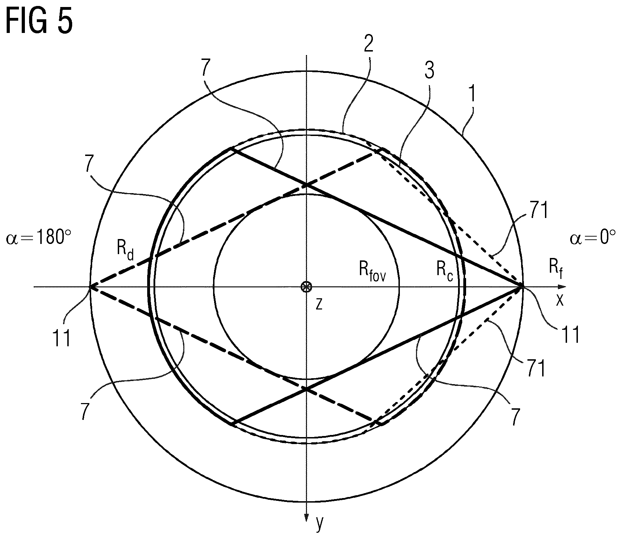

Figure 5 shows a schematic representation of an axial view of the CT ring 8. This embodiment may be the same as the one shown in figures 3 and 4. Here, two x-ray sources 11 are activated simultaneously. The two x-ray sources 11 are separated by 180° on the x-ray source array 1, i.e., in this example, one is positioned at 0° and one is positioned at 180°. In this configuration, none of the detectors 21 simultaneously receives beams from both x-ray sources 11 that both go through the examination area 4 (indicated by dashed and full lines). However, there may be some overlap with beams 71 that do not cross the examination area 4 (indicated by dotted lines).

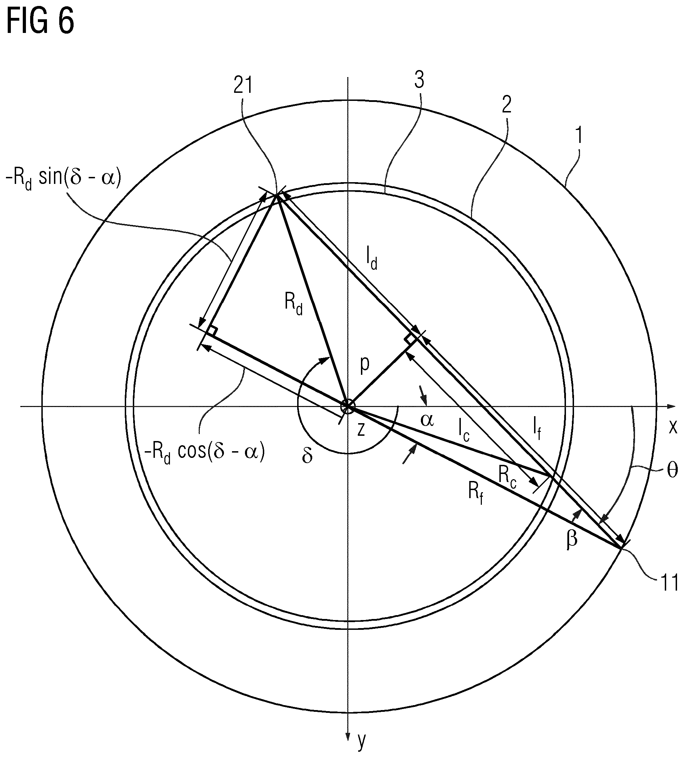

Figure 6 shows a schematic representation of an axial view of the CT ring 8 according to the embodiment shown in figure 5 while also illustrating some geometrical relations. With the geometrical relations, equations for a projection of the beam collimator 3 on the cylindrical detector arrangement 2 can be derived. Assuming a certain x-ray source 11 position ($R_f$ cos $\alpha$ , $R_f$ sin $\alpha$) and detector 21 position ($R_d$ cos $\delta$ , $R_d$ sin $\delta$), the corresponding x-ray or beam has the fan angle

$$\beta = \tan^{-1}\left(\frac{R_d\sin(\delta - \alpha)}{R_d\cos(\delta - \alpha) - R_f}\right).$$

With a parallel displacement

$$p = R_f \sin\ \beta,$$

the Pythagoras theorem yields the distances $l_f$, $l_d$, and $l_c$:

$$l_f = \sqrt{R_f^2 - p^2},\ \ l_d = \sqrt{R_d^2 - p^2}, \text{ and } l_c = \sqrt{R_c^2 - p^2}.$$

The distances $l_f$, $l_d$, and $l_c$ can be used to project the z-coordinates $z_{cl}$ and $z_{ch}$ from the collimator onto the detector 21 cylinder

$$z_{cl}' = \frac{l_f+l_d}{l_f-l_c}z_{cl},$$

$$z_{ch}' = \frac{l_f+l_d}{l_f-l_c}z_{ch}.$$

For two x-ray sources 11 that are separated by 180° (for example, as shown in figure 5, $\alpha = 0°$ and $\alpha = 180°$), the projected z-coordinates may be plotted das a function of $\delta$, i.e., the angle of the detector 21 position.

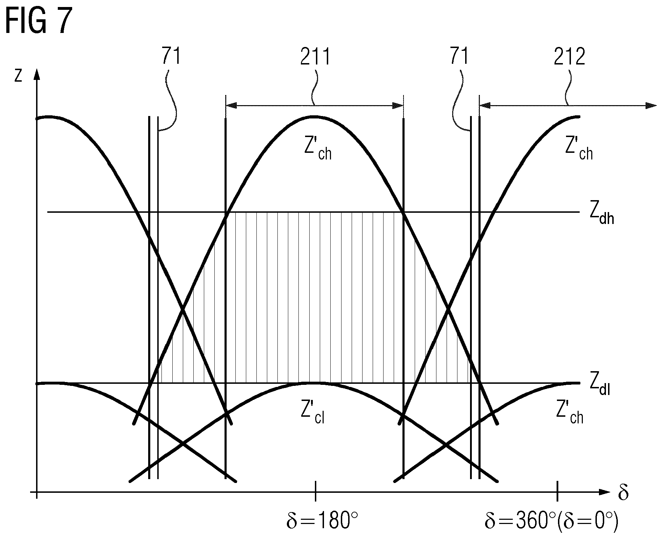

A corresponding plot is shown in figure 7. The parameters used in the plot are $R_f$= 580 mm, $R_d$= 420 mm, $R_c$= 410 mm, $R_{fov}$=250 mm, $z_{cl}$ = 2.7mm, $z_{ch}$ =7.3 mm, $z_{dl}$ = 14.3 mm, and $z_{dh}$ = 31.6 mm. Figure 7 illustrates an example of the illumination of a detector 21 by two simultaneously activated x-ray sources 11 that are separated by 180°, i.e. that are, in this example, positioned at 0° and 180°. In this example, the opening 31 of the beam collimator 3 (defined by $z_{cl}$, $z_{ch}$) is configured such that it fully illuminates the detector 21 (defined by $z_{dl}$, $z_{dh}$) for the whole scan field of view (defined by $R_{fov}$)

corresponding to the examination area 4. The curves $z'_{cl}$ and $z'_{ch}$ denote $z_{cl}$ and $z_{ch}$, respectively, being projected onto the detectors 21. Arrows indicate the projection of the scan field of view onto a first detector 211 at $\alpha=0°$ and onto a second detector 212 at $\alpha=180°$. As can be recognized in figure 7, the detector is fully illuminated within the projection of the scan field of view and partially illuminated between the projection of the scan field of view and the area defined by the dotted lines. The dotted lines correspond to the dotted lines as shown in figure 5, designating beams 71 not crossing the scan field of view. In this example, the partial illumination rage from the x-ray source 11 at 0° does not cover any part of the scan field of view projection for the x-ray source 11 at 180°, and vice versa.

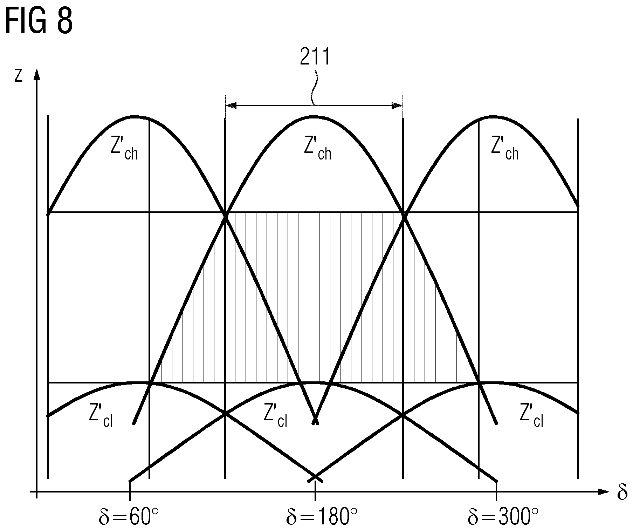

Figure 8 shows a plot similar to the one of figure 7 but for three simultaneously activated x-ray sources 11. The x-ray sources 11 are activated such that the simultaneously activated x-ray sources 11 are spaced equally on the x-ray source array 1 around the examination area 4. Hence, in this example, the spacing between the x-ray sources 11 is 120°. A scan field of view radius of $R_{fov}=243$ mm is assumed. The illumination of one of the x-ray sources 11 extends into part the detector corresponding to another one of the x-ray sources 11 (i.e., these are unwanted contributions). However, as in the example of figure 7, these unwanted contributions are not crossing the scan field of view, i.e. they are not crossing the examination area 4, and are thus unattenuated. For example, these unattenuated unwanted contributions can be measured in advance and subsequently be subtracted from the measurement. Accordingly, the actual measurement from within the examination area 4 may thus be improved.

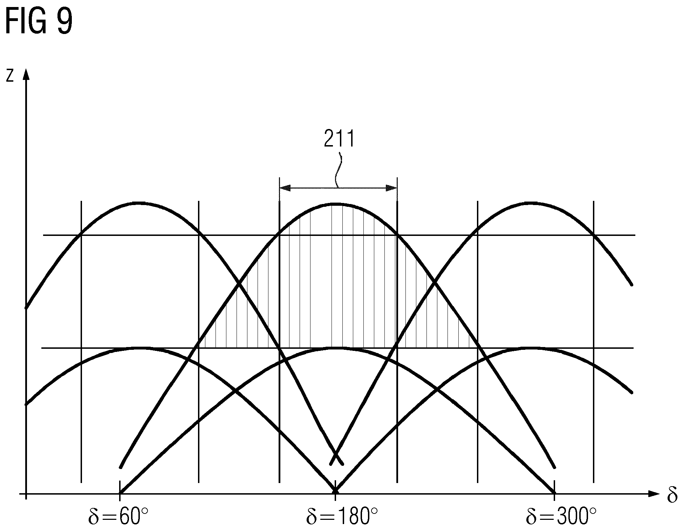

Optionally, if only a small scan field of view or a small examination area 4 is required, e.g. for a small object, the opening 31 of the beam collimator 3 may be adapted to achieve a smaller scan field of view. A corresponding plot is shown in figure 9, where parameters $R_{fov}=150$ mm, $z_{cl}=3.2$ mm, and $Z_{ch}=5.8$ mm are used. Correspondingly there is no overlap of signals within the projections of the scan field of view and a separation of the signals can be achieved more easily.

## Claims

1. Method for performing a computed tomography scan with a static computed tomography system,

   the computed tomography system comprising
   an examination area (4),
   an x-ray source array (1) with x-ray sources (11) that are arranged around the examination area (4),
   a detector arrangement (2) with detectors (21) that are arranged around the examination area (4), and
   a beam collimator (3) positioned in a radial direction between the x-ray source array (1) and the examination area (4),
   wherein the method comprises the following steps:

      acquiring data with an object or subject (5) not being in the examination area (4) to acquire unattenuated data, wherein the unattenuated data is acquired for each x-ray source (11) individually;

      (a) simultaneously activating at least two x-ray sources (11) and measuring the corresponding signals with the detector arrangement (2) in order to acquire computed tomography scan data of the object or subject (5) placed in the examination area (4), wherein the x-ray sources (11) are activated simultaneously such that none of the detectors (21) simultaneously receives beams from two different x-ray sources (11) that both go through the examination area (4);
      (b) repeating step (a) multiple times with different x-ray sources (11) of the x-ray source array (1), respectively, in order to acquire a full scan of the object or subject (5); and

      using the unattenuated data to adjust the data acquired with the object or subject (5) being in the examination area (4), wherein the adjusting is performed for each detector (21) by subtracting the unattenuated data that is contributed by one of the x-ray sources (11) and does not cross the examination area (4) before being detected by the detector (21) corresponding to another one of the x-ray sources (11) from the data acquired with the object or subject (5) being in the examination area (4).

2. The method according to claim 1,

   wherein more than two x-ray sources (11) are activated simultaneously,
   wherein the x-ray sources (11) are activated such that the simultaneously activated x-ray sources (11) are spaced equally on the x-ray source array (1) around the examination area (4).

3. The method according to claim 1,

wherein exactly two x-ray sources (11) are activated simultaneously.

4. The method according to claim 3,
wherein a smallest angle between the two simultaneously activated x-ray sources (11) on the x-ray source array (1) is smaller than 180°, preferably smaller than 176°, more preferably between 175° and 90°.

5. The method according to claim 3,
wherein an angle between the two simultaneously activated x-ray sources (11) on the x-ray source array (1) is essentially 180°.

6. A computed tomography system comprising

an examination area (4),
a static x-ray source array (1) with x-ray sources (11) that are arranged around the examination area (4),
a detector arrangement (2) with detectors (21) that are arranged around the examination area (4), and
a beam collimator (3) positioned in a radial direction between the x-ray source array (1) and the examination area (4),
wherein the computed tomography system is configured to acquire computed tomography scan data of an object or subject (5) placed in the examination area (4) by simultaneously activating at least two x-ray sources (11) and measuring the corresponding signals with the detector arrangement (2) and repeating the simultaneous activation multiple times with different x-ray sources (11) of the x-ray source array (1), respectively, in order to acquire a full scan of the object or subject (5),
wherein the computed tomography system is configured to carry out the method according to any one of claims 1 to 5.

7. The system according to claim 6,

wherein the detector arrangement (2) and the x-ray source array (1) are offset with respect to each other in an axial direction (A) perpendicular to the radial direction,
wherein an opening (31) of the beam collimator (3) is offset with respect to both, the x-ray source array (1) and the detector arrangement (2), such that it is positioned between the x-ray source array (1) and the detector arrangement (2) in the axial direction (A).

8. The system according to claim 6 or 7,
wherein the detector arrangement (2) is offset with respect to the x-ray source array (1) in the radial direction such that, in the radial direction, the detector arrangement (2) is positioned closer to the examination area (4) than the x-ray source array (1).

9. The system according to any one of claims 6 to 8,
wherein the x-ray source array (1), the x-ray sources (11) that are to be activated simultaneously, and the beam collimator (3) are arranged such that, during a scan, none of the detectors (21) simultaneously receives beams from two different x-ray sources (11) that both go through the examination area (4).

10. A computer program comprising instructions which, when the program is executed by a control unit of the computed tomography system of any one of claims 6 to 9, cause the computed tomography system to carry out the method of any one of claims 1 to 5.

## Patentansprüche

1. Verfahren zum Durchführen eines Computertomographiescans mit einem statischen Computertomographiesystem, wobei das Computertomographiesystem Folgendes umfasst:

einen Untersuchungsbereich (4),
ein Röntgenquellenarray (1) mit Röntgenquellen (11), die um den Untersuchungsbereich (4) herum angeordnet sind,
eine Detektoranordnung (2) mit Detektoren (21), die um den Untersuchungsbereich (4) herum angeordnet sind, und

einen Strahlkollimator (3), der in einer radialen Richtung zwischen dem Röntgenquellenarray (1) und dem Untersuchungsbereich (4) positioniert ist,
wobei das Verfahren ferner die folgenden Schritte umfasst:

Erfassen von Daten mit einem Objekt oder Subjekt (5), das sich nicht im Untersuchungsbereich (4) befindet, um unabgeschwächte Daten zu erfassen, wobei die unabgeschwächten Daten für jede Röntgenquelle (11) individuell erfasst werden;

(a) gleichzeitiges Aktivieren von mindestens zwei Röntgenquellen (11) und Messen der entsprechenden Signale mit der Detektoranordnung (2), um Computertomographie-Scandaten des im Untersuchungsbereich (4) platzierten Objekts oder Subjekts (5) zu erfassen, wobei die Röntgenquellen (11) gleichzeitig aktiviert werden, so dass keiner der Detektoren (21) gleichzeitig Strahlen von zwei verschiedenen Röntgenquellen (11) empfängt, die beide durch den Untersuchungsbereich (4) hindurchgehen;
(b) mehrmaliges Wiederholen von Schritt (a) mit verschiedenen Röntgenquellen (11) des Röntgenquellenarrays (1), um einen vollständigen Scan des Objekts oder Subjekts (5) zu erfassen; und

Verwenden der unabgeschwächten Daten zum Anpassen der Daten, die mit dem Objekt oder Subjekt (5), das sich im Untersuchungsbereich (4) befindet, erfasst wurden,
wobei das Anpassen für jeden Detektor (21) durch Subtrahieren der unabgeschwächten Daten, die durch eine der Röntgenquellen (11) beigetragen werden und den Untersuchungsbereich (4) nicht kreuzen, bevor sie durch den Detektor (21) detektiert werden, der einer anderen der Röntgenquellen (11) entspricht, von den Daten, die erfasst werden, wenn sich das Objekt oder Subjekt (5) in dem Untersuchungsbereich (4) befindet, durchgeführt wird.

2. Verfahren nach Anspruch 1,

wobei mehr als zwei Röntgenquellen (11) gleichzeitig aktiviert werden,
wobei die Röntgenquellen (11) so aktiviert werden, dass die gleichzeitig aktivierten Röntgenquellen (11) gleichmäßig auf dem Röntgenquellenarray (1) um den Untersuchungsbereich (4) herum beabstandet sind.

3. Verfahren nach Anspruch 1,
wobei genau zwei Röntgenquellen (11) gleichzeitig aktiviert werden.

4. Verfahren nach Anspruch 3,
wobei ein kleinster Winkel zwischen den zwei gleichzeitig aktivierten Röntgenquellen (11) auf dem Röntgenquellenarray (1) kleiner als 180°, vorzugsweise kleiner als 176 °, bevorzugter zwischen 175° und 90° ist.

5. Verfahren nach Anspruch 3,
wobei ein Winkel zwischen den zwei gleichzeitig aktivierten Röntgenquellen (11) auf dem Röntgenquellenarray (1) im Wesentlichen 180° beträgt.

6. Computertomographiesystem, umfassend:

einen Untersuchungsbereich (4),
ein statisches Röntgenquellenarray (1) mit Röntgenquellen (11), die um den Untersuchungsbereich (4) herum angeordnet sind,
eine Detektoranordnung (2) mit Detektoren (21), die um den Untersuchungsbereich (4) herum angeordnet sind, und
einen Strahlkollimator (3), der in einer radialen Richtung zwischen dem Röntgenquellenarray (1) und dem Untersuchungsbereich (4) positioniert ist,
wobei das Computertomographiesystem dazu ausgelegt ist, Computertomographie-Scandaten eines Objekts oder Subjekts (5), das in dem Untersuchungsbereich (4) platziert ist, durch gleichzeitiges Aktivieren von mindestens zwei Röntgenquellen (11) und Messen der entsprechenden Signale mit der Detektoranordnung (2) und mehrmaliges Wiederholen der gleichzeitigen Aktivierung mit verschiedenen Röntgenquellen (11) des Röntgenquellenarrays (1) zu erfassen, um einen vollständigen Scan des Objekts oder Subjekts (5) zu erfassen, wobei das Computertomographiesystem dazu ausgelegt ist, das Verfahren nach einem der Ansprüche 1 bis 5 auszuführen.

**7.** System nach Anspruch 6,

wobei die Detektoranordnung (2) und das Röntgenquellenarray (1) in einer axialen Richtung (A) senkrecht zu der radialen Richtung zueinander versetzt sind,
wobei eine Öffnung (31) des Strahlkollimators (3) bezüglich sowohl des Röntgenquellenarrays (1) als auch der Detektoranordnung (2) versetzt ist, so dass sie zwischen dem Röntgenquellenarray (1) und der Detektoranordnung (2) in der axialen Richtung (A) positioniert ist.

**8.** System nach Anspruch 6 oder 7,
wobei die Detektoranordnung (2) bezüglich des Röntgenquellenarrays (1) in der radialen Richtung versetzt ist, so dass die Detektoranordnung (2) in der radialen Richtung näher an dem Untersuchungsbereich (4) als das Röntgenquellenarray (1) positioniert ist.

**9.** System nach einem der Ansprüche 6 bis 8,
wobei das Röntgenquellenarray (1), die Röntgenquellen (11), die gleichzeitig aktiviert werden sollen, und der Strahlkollimator (3) so angeordnet sind, dass während eines Scans keiner der Detektoren (21) gleichzeitig Strahlen von zwei verschiedenen Röntgenquellen (11) empfängt, die beide durch den Untersuchungsbereich (4) hindurchgehen.

**10.** Computerprogramm, umfassend Anweisungen, die bei Ausführung des Programms durch eine Steuereinheit des Computertomographiesystems nach einem der Ansprüche 6 bis 9 bewirken, dass das Computertomographiesystem das Verfahren nach einem der Ansprüche 1 bis 5 ausführt.

**Revendications**

**1.** Procédé pour effectuer un balayage de tomodensitométrie assistée par ordinateur par un système de tomodensitométrie statique assistée par ordinateur,

le système de tomodensitométrie assistée par ordinateur comprenant une zone (4) d'examen,
un réseau (1) de sources de rayons X ayant des sources (11) de rayons X, qui sont disposées autour de la zone (4) d'examen,
un agencement (2) de détecteurs ayant des détecteurs (21), qui sont disposés autour de la zone (4) d'examen, et
un collimateur (3) de faisceaux en position dans une direction radiale entre le réseau (1) de sources de rayons X et la zone (4) d'examen,
dans lequel le procédé comprend les stades suivants :

acquérir une donnée avec un objet ou un sujet (5), qui n'est pas dans la zone (4) d'examen, pour acquérir une donnée non atténuée, la donnée non atténuée étant acquise individuellement pour chaque source (11) de rayons X ;

(a) activer simultanément au moins deux sources (11) de rayons X et mesurer les signaux correspondants par l'agencement (2) de détecteur afin d'acquérir des données de balayage de tomodensitométrie assistée par ordinateur de l'objet ou du sujet (5) placé dans la zone (4) d'examen, dans lequel les sources (11) de rayons X sont activées simultanément de manière à ce qu'aucun des détecteurs (21) ne reçoive simultanément des faisceaux de deux sources (11) de rayons X différentes, qui passent tous deux dans la zone (4) d'examen ;
(b) répéter le stade (a) de multiples fois avec des sources (11) de rayons X différentes du réseau (1) de sources de rayons X respectivement afin d'acquérir un balayage complet de l'objet ou du sujet (5) ; et

utiliser la donnée non atténuée pour ajuster la donnée acquise alors que l'objet ou le sujet (5) est dans la zone (4) d'examen, dans lequel l'ajustement est effectué pour chaque détecteur (21) en soustrayant la donnée non atténuée, qui est donnée par l'une des sources (11) de rayons X et ne traverse pas la zone (4) d'examen avant d'être détectée par le détecteur (21) correspondant à une autre des sources (11) de rayons X de la donnée acquise alors que l'objet ou le sujet (5) est dans la zone (4) d'examen.

**2.** Le procédé suivant la revendication 1,

dans lequel on active simultanément plus de deux sources (11) de rayons X,

dans lequel on active les sources (11) de rayons X de manière à ce que les sources (11) de rayons X activées simultanément soient réparties également sur le réseau (1) de sources de rayons X autour de la zone (4) d'examen.

3. Le procédé suivant la revendication 1,
dans lequel on active simultanément exactement deux sources (11) de rayons X.

4. Le procédé suivant la revendication 3,
dans lequel l'angle le plus petit entre les deux sources (11) de rayons X activées simultanément sur le réseau (1) de sources de rayons X est plus petit que 180°, de préférence plus petit que 176°, d'une manière plus préférée compris entre 175° et 90°.

5. Le procédé suivant la revendication 3,
dans lequel un angle entre les deux sources (11) de rayons X activées simultanément sur le réseau (1) de sources de rayons X est essentiellement de 180°.

6. Un système de tomodensitométrie assistée par ordinateur comprenant

une zone (4) d'examen,
un réseau (1) statique de sources de rayons X ayant des sources (11) de rayons X, qui sont disposées autour de la zone (4) d'examen,
un agencement (2) de détecteurs ayant des détecteurs (21), qui sont disposés autour de la zone (4) d'examen, et
un collimateur (3) de faisceaux en position dans une direction radiale entre le réseau (1) de sources de rayons X et la zone (4) d'examen,
dans lequel le système de tomodensitométrie assistée par ordinateur est configuré pour acquérir des données de balayage de tomodensitométrie assistée par ordinateur d'un objet ou d'un sujet (5) placé dans la zone (4) d'examen en activant simultanément au moins deux sources (11) de rayons X et en mesurant les signaux correspondants par l'agencement (2) de détecteurs et en répétant l'activation simultanée de multiples fois avec des sources (11) de rayons X différentes de réseau (1) de sources de rayons X, respectivement, afin d'acquérir un balayage complet de l'objet ou du sujet (5),
dans lequel le système de tomodensitométrie assistée par ordinateur est configuré pour effectuer le procédé suivant l'une quelconque des revendications 1 à 5.

7. Le système suivant la revendication 6,
dans lequel l'agencement (2) de détecteurs et le réseau (1) de sources de rayons X sont décalés l'un par rapport à l'autre dans une direction (A) axiale perpendiculaire à la direction radiale, dans lequel une ouverture (31) du collimateur (3) de faisceaux est décalée par rapport aux deux, le réseau (1) de sources de rayons X et l'agencement (2) de détecteurs, de manière à être en position entre le réseau (1) de sources de rayons X et l'agencement (2) de détecteurs dans la direction (A) axiale.

8. Le système suivant la revendication 6 ou 7,
dans lequel l'agencement (2) de détecteurs est décalé par rapport au réseau (1) de sources de rayons X dans la direction radiale de manière à ce que, dans la direction radiale, l'agencement (2) de détecteurs soit en position plus près de la zone (4) d'examen que du réseau (1) de sources de rayons X.

9. Le système suivant l'une quelconque des revendications 6 à 8,
dans lequel le réseau (1) de sources de rayons X, les sources (11) de rayons X à activer simultanément et le collimateur (3) de faisceaux sont disposés de manière à ce que, pendant un balayage, aucun détecteur (21) ne reçoive simultanément des faisceaux de deux sources (11) de rayons X différentes, qui passent tous deux dans la zone (4) d'examen.

10. Un programme d'ordinateur comprenant des instructions qui, lorsque le programme est exécuté par une unité de commande du système de tomodensitométrie assistée par ordinateur suivant l'une quelconque des revendications 6 à 9, font que le système de tomodensitométrie assistée par ordinateur effectue le procédé suivant l'une quelconque des revendications 1 à 5.

FIG 1

FIG 2

## FIG 3

## FIG 4

FIG 5

# FIG 6

# FIG 7

FIG 8

FIG 9

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20210383582 A1

**Non-patent literature cited in the description**

- **FELDKAMP, I.** ; **DAVIS, L.** ; **KRESS**. *J. Practical cone-beam algorithm Journal of the Optical Society of America A: Optics and Image Science, and Vision*, 1984, vol. 1, 612-619

- **KUNZE, H.** ; **HÄRER, H.** ; **STIERSTORFER K.** Iterative extended field of view reconstruction. *Proceedings of SPIE - The International Society for Optical Engineering*, 2007